# EUROPEAN PATENT SPECIFICATION

(11) **EP 1 308 524 B1**
(45) Date of publication and mention of the grant of the patent: **31.05.2006**
(21) Application number: 02022399.6
(22) Date of filing: 10.10.2002
(51) Int. Cl.: C12Q 1/70

(54) **Detection of variola virus**
Nachweis von Variola Virus
Procédé de détection du virus de la variole

(30) Priority: 02.11.2001 US 338237 P; 17.12.2001 US 341601 P
(43) Date of publication of application: 07.05.2003
(73) Proprietor: Roche Diagnostics GmbH, 82377 Penzberg (DE); Mayo Foundation for Medical Education and Research, Rochester, MN 55905-0001 (US)
(72) Inventor: Smith, Thomas F., Rochester, MN 55902 (US); Espy, Mark J., Rochester, MN 55906 (US)

(56) References cited:
- DATABASE EMBL [Online] 4 March 2000 (2000-03-04), MASSUNG ET AL.: "potential virulence determinants in terminal regions of variola smallpox virus genome" XP002261821 accession no. EBI Database accession no. L22579 & NATURE, vol. 366, no. 6457, 1993, pages 748-751,
- ROPP S L ET AL: "PCR strategy for identification and differentiation of small pox and other orthopoxviruses." JOURNAL OF CLINICAL MICROBIOLOGY. UNITED STATES AUG 1995, vol. 33, no. 8, August 1995 (1995-08), pages 2069-2076, XP002261862 ISSN: 0095-1137
- ESPY M J ET AL: "Diagnosis of varicella-zoster virus infections in the clinical laboratory by LightCycler PCR." JOURNAL OF CLINICAL MICROBIOLOGY. UNITED STATES SEP 2000, vol. 38, no. 9, September 2000 (2000-09), pages 3187-3189, XP002261863 ISSN: 0095-1137
- VAN ELDEN L J ET AL: "Simultaneous detection of influenza viruses A and B using real-time quantitative PCR." JOURNAL OF CLINICAL MICROBIOLOGY. UNITED STATES JAN 2001, vol. 39, no. 1, January 2001 (2001-01), pages 196-200, XP002261864 ISSN: 0095-1137
- MEYER H ET AL: "Gene for A-type inclusion body protein is useful for a polymerase chain reaction assay to differentiate orthopoxviruses." JOURNAL OF VIROLOGICAL METHODS. NETHERLANDS MAR 1997, vol. 64, no. 2, March 1997 (1997-03), pages 217-221, XP002261865 ISSN: 0166-0934
- ESPY ET AL: "Diagnosis of Herpes Simplex Virus Infections in the Clinical Laboratory by LightCycler PCR" JOURNAL OF CLINICAL MICROBIOLOGY, WASHINGTON, DC, US, vol. 38, no. 2, February 2000 (2000-02), pages 795-799, XP002175791 ISSN: 0095-1137
- RYNCARZ A J ET AL: "Development of a high-throughput quantitative assay for detecting herpes simplex virus DNA in clinical samples" JOURNAL OF CLINICAL MICROBIOLOGY, WASHINGTON, DC, US, vol. 37, no. 6, June 1999 (1999-06), pages 1941-1947, XP002247593 ISSN: 0095-1137
- ESPY MARK J ET AL: "Detection of smallpox virus DNA by LightCycler PCR." JOURNAL OF CLINICAL MICROBIOLOGY. UNITED STATES JUN 2002, vol. 40, no. 6, June 2002 (2002-06), pages 1985-1988, XP002261866 ISSN: 0095-1137
- DATABASE EMBL [Online] 13 December 1996 (1996-12-13), SHELKUNOV ET AL.: "variola virus DNA complete genome" XP002261992 accession no. EBI Database accession no. X69198

## Description

### TECHNICAL FIELD

This invention relates to viral diagnostics, and more particularly to detection of variola virus.

### PRIOR ART BACKGROUND

Routine immunization in the U.S. was discontinued in 1971 because the risk of disseminated infection in infants with immunodeficiency conditions outweighed the likelihood of contracting subsequent smallpox infection. The last case of smallpox occurred in 1977 in Somalia, Africa. In 1980, the World Health Organization (WHO) declared that smallpox had been eradicated. As a result of this policy, 30 years have past yielding a young population who are likely susceptible to smallpox virus infection. Use of smallpox as a component of biological warfare remains a threat for producing epidemic disease in those individuals never immunized against this virus and perhaps adults who received the vaccine in the remote past.

Detection of smallpox by PCR has been done using primers based on genome sequences encoding the hemagglutinin (HA) protein. A set of primers specific for variola virus has been disclosed (Ropp et al., J. Clinical Microbiology, vol 33, pages 2069-2076).

### BRIEF DESCRIPTION OF THE INVENTION

The invention provides for methods of identifying variola in a biological sample. Primers and probes for detecting variola virus are provided by the invention, as are kits containing such primers and probes. Methods of the invention can be used to rapidly identify variola virus DNA from specimens for diagnosis of variola virus infection and to differentiate variola virus infections from HSV infections. Using specific primers and probes, the methods include amplifying and monitoring the development of specific amplification products using fluorescence resonance energy transfer (FRET).

In one aspect of the invention, there is provided a method for detecting the presence or absence of variola virus in a biological sample from an individual. The method to detect variola virus includes performing at least one cycling step, which includes an amplifying step and a hybridizing step. The amplifying step includes contacting the sample with a pair of hemagglutinin (HA) primers to produce an HA amplification product if a variola virus HA nucleic acid molecule is present in the sample. The hybridizing step includes contacting the sample with a pair of HA probes. Generally, the members of the pair of HA probes hybridize within no more than five nucleotides of each other. A first HA probe of the pair of HA probes is typically labeled with a donor fluorescent moiety and a second HA probe of the pair of HA probes is labeled with a corresponding acceptor fluorescent moiety. The method further includes detecting the presence or absence of FRET between the donor fluorescent moiety of the first HA probe and the acceptor fluorescent moiety of the second HA probe. The presence of FRET is usually indicative of the presence of variola virus in the biological sample, while the absence of FRET is usually indicative of the absence of variola virus in the biological sample.

the amplifying step further includes contacting the sample with a pair of thymidine kinase (TK) primers to produce a TK amplification product if a variola virus TK nucleic acid molecule is present in the sample. The hybridizing step includes contacting the sample with a pair of TK probes. Generally, the members of the pair of TK probes hybridize within no more than five nucleotides of each other. A first TK probe of the pair of TK probes is typically labeled with a donor fluorescent moiety and a second TK probe of the pair of TK probes is labeled with a corresponding acceptor fluorescent moiety. The method further includes detecting the presence or absence of FRET between the donor fluorescent moiety of the first TK probe and the acceptor fluorescent moiety of the second TK probe. The presence of FRET is usually indicative of the presence of variola virus in the biological sample, while the absence of FRET is usually indicative of the absence of variola virus in the biological sample. The methods to detect variola virus using HA and TK can be performed sequentially or concurrently.

Primers and probes according to the invention can be directed to a first portion (HA1) or a second portion (HA2) of the HA gene. A pair of HA1 primers generally includes a first HA1 primer and a second HA1 primer. The first HA1 primer can include the sequence 5'-CTA ATA TCA TTA GTA TAC GCT ACA C-3' (SEQ ID NO: 1), and the second HA1 primer can include the sequence 5'-GAG TCG TAA GAT ATT TTA TCC-3' (SEQ ID NO:2). A first HA1 probe can include the sequence 5'-AAT GAT TAT GTT GTT ATG AGT GCT TG-3' (SEQ ID NO:3) and the second HA1 probe can include the sequence 5'-TAT AAG GAG CCC AAT TCC ATT ATT CT-3' (SEQ ID NO:4).

A pair of HA2 primers generally includes a first HA2 primer and a second HA2 primer. The first HA2 primer can include the sequence 5'-ATA GTG AAT CGA CTA TAG ACA TAA TA-3' (SEQ ID NO: 5), and the second HA2 primer can include the sequence 5'-TTG ATT TAG TAG TGA CAA TTC C-3' (SEQ ID NO:6). A first HA2 probe can include the sequence 5'-CTG TCA CAT ACA CTA GTG ATA TCA TT-3' (SEQ ID NO:7), and the second HA2 probe can include the sequence 5'-ATA CAG TAA GTA CAT CAT CTG GAG AA-3' (SEQ ID NO:8).

A pair of TK primers generally includes a first TK primer and a second TK primer. The first TK primer can include the sequence 5'-AAG GAC AGT TCT TTC CAG-3' (SEQ ID NO:9), and the second TK primer can include the sequence 5'-TGA TAC ATA TCA TTA CCT CCT A-3' (SEQ ID NO: 10). A first TK probe can include the sequence 5'-CCG TTT AAT AAT ATC TTG GAT CTT-3' (SEQ ID NO: 11), and the second TK probe can include the sequence 5'-TTC CAT TAT CTG AAA TGG TGG T-3' (SEQ ID NO:12). Alternatively or additionally, a second pair of TK probes having the following sequences can be used to detect a TK amplification product: 5'-GAC ATT TCA ACG TAA ACC GTT TAA-3' (SEQ ID NO:13); and 5'-AATATC TTG GAT CTT ATT CCA TTA TCT G-3' (SEQ ID NO: 14). In some aspects, one of the HA or TK primers can be labeled with a fluorescent moiety (either a donor or acceptor, as appropriate) and can take the place of the HA or TK probes, respectively.

The members of the pair of HA probes or TK probes can hybridize within no more than two nucleotides of each other, or can hybridize within no more than one nucleotide of each other. A representative donor fluorescent moiety is fluorescein, and corresponding acceptor fluorescent moietes include LC-Red 640, LC-Red 705, Cy5, and Cy5.5. Additional corresponding donor and acceptor fluorescent moieties are known in the art.

In one aspect, the detecting step includes exciting the biological sample at a wavelength absorbed by the donor fluorescent moiety and visualizing and/or measuring the wavelength emitted by the acceptor fluorescent moiety (*i.e*., visualizing and'/or measuring FRET). In another aspect, the detecting step includes quantitating the FRET. In yet another aspect, the detecting step can be performed after each cycling step *(e.g.,* in real-time).

Generally, the presence of FRET within 50 cycles (*e.g.,* 20, 25, 30, 35, 40, or 45 cycles) indicates the presence of a variola virus infection in the individual. In addition, determining the melting temperature between one or both of the HA probe(s) and the HA amplification or, similarly, one or both of the TK probe(s) and the TK amplification product can confirm the presence or absence of the variola virus.

Representative biological sample include dermal swabs, cerebrospinal fluid, ganglionic tissue, brain tissue, ocular fluid, blood, sputum, bronchio-alveolar lavage, bronchial aspirates, lung tissue, and urine. The above-described methods can further include preventing amplification of a contaminant nucleic acid. Preventing amplification can include performing the amplifying step in the presence of uracil and treating the biological sample with uracil-DNA glycosylase prior to amplifying.

In addition, the cycling step can be performed on a control sample. A control sample can include the same portion of the variola virus HA nucleic acid molecule. Alternatively, a control sample can include a nucleic acid molecule other than a variola virus HA nucleic acid molecule. Cycling steps can be performed on such a control sample using a pair of control primers and a pair of control probes. The control primers and probes are other than HA primers and probes. One or more amplifying steps produces a control amplification product. Each of the control probes hybridizes to the control amplification product.

In another aspect, there are provided articles of manufacture, or kits. Kits of the invention can include a pair of HA primers (HA1 or HA2), and a pair of HA probes (HA1 or HA2, respectively), and a donor and corresponding acceptor fluorescent moieties. For example, a first HA1 primer provided in a kit of the invention can have the sequence 5'-CTA ATA TCA TTA GTA TAC GCT ACA C-3' (SEQ ID NO:1) and a second HA1 primer can have the sequence 5'-GAG TCG TAA GAT ATT TTA TCC-3' (SEQ ID NO:2). A first HA1 probe provided in a kit of the invention can have the sequence 5'-AAT GAT TAT GTT GTT ATG AGT GCT TG-3' (SEQ ID NO:3) and a second HA1 probe can have the sequence 5'-TAT AAG GAG CCC AAT TCC ATT ATT CT-3' (SEQ ID NO:4). A first HA2 primer provided in a kit of the invention can have the sequence 5'-ATA GTG AAT CGA CTA TAG ACA TAA TA-3' (SEQ ID NO:5) and a second HA2 primer can have the sequence 5'-TTG ATT TAG TAG TGA CAA TTC C-3' (SEQ ID NO:6). A first HA2 probe provided in a kit of the invention can have the sequence 5'-CTG TCA CAT ACA CTA GTG ATA TCA TT-3' (SEQ ID NO:7) and a second HA2 probe can have the sequence 5'-ATA CAG TAA GTA CAT CAT CTG GAG AA-3' (SEQ ID NO:8).

Articles of manufacture can further or alternatively include a pair of TK primers, a pair of TK probes, and a donor and corresponding acceptor fluorescent moieties. For example, the first TK primer provided in a kit of the invention can have the sequence 5'-AAG GAC AGT TCT TTC CAG-3' (SEQ ID NO:9), and the second TK primer can have the sequence 5'-TGA TAC ATA TCA TTA CCT CCT A-3' (SEQ ID NO: 10). The first TK probe provided in a kit of the invention can have the sequence 5'-CCG TTT AAT AAT ATC TTG GAT CTT-3' (SEQ ID NO: 11), and the second TK probe can have the sequence 5'-TTC CAT TAT CTG AAA TGG TGG T-3' (SEQ ID NO:12). Alternatively, the following TK probes can be provided in a kit of the invention: 5'-GAC ATT TCA ACG TAA ACC GTT TAA-3' (SEQ ID NO:13) and 5'-AAT ATC TTG GAT CTT ATT CCA TTA TCT G-3' (SEQ ID NO: 14).

Articles of manufacture can include fluorophoric moieties for labeling the probes or probes already labeled with donor and corresponding acceptor fluorescent moieties. The article of manufacture can also include a package insert having instructions thereon for using the primers, probes, and fluorophoric moieties to detect the presence or absence of variola virus in a biological sample.

In yet another aspect, there is provided a method for detecting the presence or absence of variola virus in a biological sample from an individual. Such a method includes performing at least one cycling step. A cycling step can include an amplifying step and a hybridizing step. Generally, an amplifying step includes contacting the sample with a pair of HA primers to produce an HA amplification product if a variola virus HA nucleic acid molecule is present in the sample. Generally, a hybridizing step includes contacting the sample with an HA probe to the HA amplification product. Such an HA probe is usually labeled with a donor fluorescent moiety and a corresponding acceptor fluorescent moiety. The method further includes detecting the presence or absence of fluorescence resonance energy transfer (FRET) between the donor fluorescent moiety and the acceptor fluorescent moiety of the HA probe. The presence or absence of fluorescence is indicative of the presence or absence of variola virus in said sample. In addition to the HA primers/probe described herein, this method also can be performed using TK primers/probe.

In one aspect, amplification can employ a polymerase enzyme having 5' to 3' exonuclease activity. Thus, the first and second fluorescent moieties would be within no more than 5 nucleotides of each other along the length of the probe. In another aspect, the HA probe includes a nucleic acid sequence that permits secondary structure formation. Such secondary structure formation generally results in spatial proximity between the first and second fluorescent moiety. According to this method, the second fluorescent moiety on a probe can be a quencher.

In another aspect , there is provided a method for detecting the presence or absence of variola virus in a biological sample from an individual. Such a method includes performing at least one cycling step. A cycling step can include an amplifying step and a dye-binding step. An amplifying step generally includes contacting the sample with a pair of HA primers to produce an HA amplification product if a variola virus HA nucleic acid molecule is present in the sample. A dye-binding step generally includes contacting the HA amplification product with a double-stranded DNA binding dye. The method further includes detecting the presence or absence of binding of the double-stranded DNA binding dye, the presence of binding is typically indicative of the presence of variola virus in the sample, and the absence of binding is typically indicative of the absence of variola virus in the sample. Such a method can further include the steps of determining the melting temperature between the HA amplification product and the double-stranded DNA binding dye. Generally, the melting temperature confirms the presence or absence of variola virus. Representative double-stranded DNA binding dyes include SYBRGreenI® , SYBRGold® , and ethidium bromide.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In addition, the materials, methods, and examples are illustrative only and not intended to be limiting. All publications, patent applications, patents, and other references mentioned herein are incorporated by reference in their entirety. In case of conflict, the present specification, including definitions, will control.

The details of one or more embodiments of the invention are set forth in the accompanying drawings and the description below. Other features, objects, and advantages of the invention will be apparent from the drawings and detailed description, and from the claims.

### DETAILED DESCRIPTION

A real-time assay for detecting variola virus in a biological sample that is more sensitive than existing assays is described herein. Primers and probes for detecting variola virus infections and articles of manufacture containing such primers and probes are provided by the invention. The increased sensitivity of real-time PCR for detection of variola virus compared to other methods, as well as the improved features of real-time PCR including sample containment and real-time detection of the amplified product, make feasible the implementation of this technology for routine diagnosis of variola virus infections in the clinical laboratory.

### Variola virus

Variola (smallpox) is a linear, double-stranded DNA-containing virus in the *Poxviridae* family. Variola virions are large (~300 x 250 nm), brick-shaped particles. Clinically, vesicular lesions occur in susceptible individuals after an incubation period of 8-10 days after exposure to smallpox virus. Vesicular lesions produced by herpes simplex virus (HSV) and varicella-zoster virus (VZV) would be considered in the differential clinical diagnosis of smallpox virus infection. Vaccinia virus, closely related antigenically and genomically (>95% nucleotide identity) to cowpox and smallpox viruses, was incorporated into an attenuated live smallpox vaccine.

### Variola virus nucleic acids and oligonucleotides

The invention provides methods to detect variola virus by amplifying, for example, a portion of the variola virus hemagglutinin (HA) or thymidine kinase (TK) nucleic acid. Variola virus nucleic acids other than those exemplified herein (*e.g*., other than HA and TK) also can be used to detect variola virus in a sample and are known to those of skill in the art. The nucleic acid sequence of the variola virus genome, as well as variola nucleic acid molecules encoding HA and TK, are available (see, for example, GenBank Accession Nos. M14783, and K02031). Specifically, primers and probes to amplify and detect variola virus HA nucleic acid molecules are provided by the invention, as are primers and probes to amplify and detect variola virus TK nucleic acid molecules.

Primers that amplify a variola virus nucleic acid molecule, *e.g*., variola virus HA or TK, can be designed using, for example, a computer program such as OLIGO (Molecular Biology Insights, Inc., Cascade, CO). Important features when designing oligonucleotides to be used as amplification primers include, but are not limited to, an appropriate size amplification product to facilitate detection (*e.g*., by electrophoresis), similar melting temperatures for the members of a pair of primers, and the length of each primer (*i.e*., the primers need to be long enough to anneal with sequence-specificity and to initiate synthesis but not so long that fidelity is reduced during oligonucleotide synthesis). Typically, oligonucleotide primers are 15 to 30 nucleotides in length.

Designing oligonucleotides to be used as hybridization probes can be performed in a manner similar to the design of primers, although the members of a pair of probes preferably anneal to an amplification product within no more than 5 nucleotides of each other on the same strand such that FRET can occur (*e.g.,* within no more than 1, 2, 3, or 4 nucleotides of each other). This minimal degree of separation typically brings the respective fluorescent moieties into sufficient proximity such that FRET occurs. It is to be understood, however, that other separation distances (*e.g*., 6 or more nucleotides) are possible provided the fluorescent moieties are appropriately positioned relative to each other (for example, with a linker arm) such that FRET can occur. In addition, probes can be designed to hybridize to targets that contain a polymorphism or mutation, thereby allowing differential detection of variola virus strains based on either absolute hybridization of different pairs of probes corresponding to the particular variola virus strain to be distinguished or differential melting temperatures between, for example, members of a pair of probes and each amplification product corresponding to a variola virus strain to be distinguished. As with oligonucleotide primers, oligonucleotide probes usually have similar melting temperatures, and the length of each probe must be sufficient for sequence-specific hybridization to occur but not so long that fidelity is reduced during synthesis. Oligonucleotide probes are generally 15 to 30 nucleotides in length.

Constructs include vectors containing a variola virus nucleic acid molecule, *e.g.,* variola virus HA or TK, or fragment thereof. Constructs of the invention can be used, for example, as control template nucleic acid molecules. Vectors suitable for use in the present invention are commercially available and/or produced by recombinant DNA technology methods routine in the art. Variola virus HA or TK nucleic acid molecules can be obtained, for example, by chemical synthesis, direct cloning from variola virus, or by PCR amplification. A variola virus nucleic acid molecule or fragment thereof can be operably linked to a promoter or other regulatory element such as an enhancer sequence, a response element, or an inducible element that modulates expression of the variola virus nucleic acid molecule. As used herein, operably linking refers to connecting a promoter and/or other regulatory elements to a variola virus nucleic acid molecule in such a way as to permit and/or regulate expression of the variola virus nucleic acid molecule. A promoter that does not normally direct expression of variola virus HA or TK can be used to direct transcription of an HA or TK nucleic acid using, for example, a viral polymerase, a bacterial polymerase, or a eukaryotic RNA polymerase II. Alternatively, the HA or TK native promoter can be used to direct transcription of an HA or TK nucleic acid, respectively, using, for example, a variola virus RNA polymerase enzyme. In addition, operably linked can refer to an appropriate connection between a variola virus HA or TK promoter or regulatory element and a heterologous coding sequence (*i.e*., a non-HA or -TK coding sequence, for example, a reporter gene) in such a way as to permit expression of the heterologous coding sequence.

Constructs suitable for use in the methods of the invention typically include, in addition to variola virus HA or TK nucleic acid molecules, sequences encoding a selectable marker *(e.g.,* an antibiotic resistance gene) for selecting desired constructs and/or transformants, and an origin of replication. The choice of vector systems usually depends upon several factors, including, but not limited to, the choice of host cells, replication efficiency, selectability, inducibility, and the ease of recovery.

Constructs containing variola virus HA or TK nucleic acid molecules can be propagated in a host cell. As used herein, the term host cell is meant to include prokaryotes and eukaryotes such as yeast, plant and animal cells. Prokaryotic hosts may include *E. coli, Salmonella typhimurium, Serratia marcescens* and *Bacillus subtilis*. Eukaryotic hosts include yeasts such as *S. cerevisiae, S. pombe, Pichia pastoris*, mammalian cells such as COS cells or Chinese hamster ovary (CHO) cells, insect cells, and plant cells such as *Arabidopsis thaliana* and *Nicotiana tabacum*. A construct can be introduced into a host cell using any of the techniques commonly known to those of ordinary skill in the art. For example, calcium phosphate precipitation, electroporation, heat shock, lipofection, microinjection, and viral-mediated nucleic acid transfer are common methods for introducing nucleic acids into host cells. In addition, naked DNA can be delivered directly to cells (see, *e.g.,* U.S. Patent Nos. 5,580,859 and 5,589,466).

### Polymerase chain reaction (PCR)

U.S. Patent Nos. 4,683,202, 4,683,195, 4,800,159, and 4,965,188 disclose conventional PCR techniques. PCR typically employs two oligonucleotide primers that bind to a selected nucleic acid template *(e.g.,* DNA or RNA). Primers useful in the present invention include oligonucleotides capable of acting as a point of initiation of nucleic acid synthesis within variola virus HA or TK. A primer can be purified from a restriction digest by conventional methods, or it can be produced synthetically. The primer is preferably single-stranded for maximum efficiency in amplification, but the primer can be double-stranded. Double-stranded primers are first denatured, *i.e*., treated to separate the strands. One method of denaturing double stranded nucleic acids is by heating.

The term "thermostable polymerase" refers to a polymerase enzyme that is heat stable, *i.e.,* the enzyme catalyzes the formation of primer extension products complementary to a template and does not irreversibly denature when subjected to the elevated temperatures for the time necessary to effect denaturation of double-stranded template nucleic acids. Generally, the synthesis is initiated at the 3' end of each primer and proceeds in the 5' to 3' direction along the template strand. Thermostable polymerases have been isolated from *Thermus flavus, T. ruber, T. thermophilus, T. aquaticus, T. lacteus, T. rubens, Bacillus stearothermophilus*, and *Methanothermus fervidus*. Nonetheless, polymerases that are not thermostable also can be employed in PCR assays provided the enzyme is replenished.

If the variola virus template nucleic acid is double-stranded, it is necessary to separate the two strands before it can be used as a template in PCR. Strand separation can be accomplished by any suitable denaturing method including physical, chemical or enzymatic means. One method of separating the nucleic acid strands involves heating the nucleic acid until it is predominately denatured (*e.g.,* greater than 50%, 60%, 70%, 80%, 90% or 95% denatured). The heating conditions necessary for denaturing template nucleic acid will depend, *e.g.,* on the buffer salt concentration and the length and nucleotide composition of the nucleic acids being denatured, but typically range from about 90°C to about 105°C for a time depending on features of the reaction such as temperature and the nucleic acid length. Denaturation is typically performed for about 30 sec to 4 min.

If the double-stranded nucleic acid is denatured by heat, the reaction mixture is allowed to cool to a temperature that promotes annealing of each primer to its target sequence on the variola virus nucleic acid. The temperature for annealing is usually from about 35°C to about 65°C. The reaction mixture is then adjusted to a temperature at which the activity of the polymerase is promoted or optimized, *i.e.,* a temperature sufficient for extension to occur from the annealed primer to generate products complementary to the template nucleic acid. The temperature should be sufficient to synthesize an extension product from each primer that is annealed to a nucleic acid template, but should not be so high as to denature an extension product from its complementary template (*e.g*., the temperature generally ranges from about 40° to 80°C).

PCR assays can employ variola virus nucleic acid such as DNA or RNA, including messenger RNA (mRNA). The template nucleic acid need not be purified; it may be a minor fraction of a complex mixture, such as variola virus nucleic acid contained in human cells. DNA or RNA may be extracted from a biological sample such as dermal swabs, cerebrospinal fluid, ganglionic tissue, brain tissue, ocular fluid, blood, sputum, bronchio-alveolar lavage, bronchial aspirates, lung tissue, and urine by routine techniques such as those described in *Diagnostic Moleculear Microbiology: Principles and Applications* (Persing et al. (eds), 1993, American Society for Microbiology, Washington D.C). Nucleic acids can be obtained from any number of sources, such as plasmids, or natural sources including bacteria, yeast, viruses, organelles, or higher organisms such as plants or animals.

The oligonucleotide primers are combined with PCR reagents under reaction conditions that induce primer extension. For example, chain extension reactions generally include 50 mM KCl, 10 mM Tris-HCl (pH 8.3), 15 mM MgCl₂, 0.001% (w/v) gelatin, 0.5-1.0 ^{c}cg denatured template DNA, 50 pmoles of each oligonucleotide primer, 2.5 U of Taq polymerase, and 10% DMSO). The reactions usually contain 150 to 320 ^{c}cM each of dATP, dCTP, dTTP, dGTP, or one or more analogs thereof.

The newly synthesized strands form a double-stranded molecule that can be used in the succeeding steps of the reaction. The steps of strand separation, annealing, and elongation can be repeated as often as needed to produce the desired quantity of amplification products corresponding to the target variola virus nucleic acid molecule. The limiting factors in the reaction are the amounts of primers, thermostable enzyme, and nucleoside triphosphates present in the reaction. The cycling steps (*i.e*., denaturation, annealing, and extension) are preferably repeated at least once. For use in detection, the number of cycling steps will depend, *e.g*., on the nature of the sample. If the sample is a complex mixture of nucleic acids, more cycling steps will be required to amplify the target sequence sufficient for detection. Generally, the cycling steps are repeated at least about 20 times, but may be repeated as many as 40, 60, or even 100 times.

### Fluorescence resonance energy transfer (FRET)

FRET technology (see, for example, U.S. Patent Nos. 4,996,143, 5,565,322, 5,849,489, and 6,162,603) is based on a concept that when a donor and a corresponding acceptor fluorescent moiety are positioned within a certain distance of each other, energy transfer takes place between the two fluorescent moieties that can be visualized or otherwise detected and/or quantitated. Two oligonucleotide probes, each containing a fluorescent moiety, can hybridize to an amplification product at particular positions determined by the complementarity of the oligonucleotide probes to the variola virus target nucleic acid sequence. Upon hybridization of the oligonucleotide probes to the amplification product nucleic acid at the appropriate positions, a FRET signal is generated.

Fluorescent analysis can be carried out using, for example, a photon counting epifluorescent microscope system (containing the appropriate dichroic mirror and filters for monitoring fluorescent emission at the particular range), a photon counting photomultiplier system or a fluorometer. Excitation to initiate energy transfer can be carried out with an argon ion laser, a high intensity mercury (Hg) arc lamp, a fiber optic light source, or other high intensity light source appropriately filtered for excitation in the desired range.

As used herein with respect to donor and corresponding acceptor fluorescent moieties "corresponding" refers to an acceptor fluorescent moiety having an emission spectrum that overlaps the excitation spectrum of the donor fluorescent moiety. The wavelength maximum of the emission spectrum of the acceptor fluorescent moiety should be at least 100 nm greater than the wavelength maximum of the excitation spectrum of the donor fluorescent moiety. Accordingly, efficient non-radiative energy transfer can be produced therebetween.

Fluorescent donor and corresponding acceptor moieties are generally chosen for (a) high efficiency Förster energy transfer; (b) a large final Stokes shift (>100 nm); (c) shift of the emission as far as possible into the red portion of the visible spectrum (>600 nm); and (d) shift of the emission to a higher wavelength than the Raman water fluorescent emission produced by excitation at the donor excitation wavelength. For example, a donor fluorescent moiety can be chosen that has its excitation maximum near a laser line (for example, Helium-Cadmium 442 nm or Argon 488 nm), a high extinction coefficient, a high quantum yield, and a good overlap of its fluorescent emission with the excitation spectrum of the corresponding acceptor fluorescent moiety. A corresponding acceptor fluorescent moiety can be chosen that has a high extinction coefficient, a high quantum yield, a good overlap of its excitation with the emission of the donor fluorescent moiety, and emission in the red part of the visible spectrum (>600 nm).

Representative donor fluorescent moieties that can be used with various acceptor fluorescent moieties in FRET technology include fluorescein, Lucifer Yellow, B-phycoerythrin, 9-acridineisothiocyanate, Lucifer Yellow VS, 4-acetamido-4'-isothio-cyanatostilbene-2,2'-disulfonic acid, 7-diethylamino-3-(4'-isothiocyanatophenyl)-4-methylcoumarin, succinimdyl 1-pyrenebutyrate, and 4-acetamido-4'-isothiocyanatostilbene-2,2'-disulfonic acid derivatives. Representative acceptor fluorescent moieties, depending upon the donor fluorescent moiety used, include LC^{™-}Red 640, LC^{™}-Red 705, Cy5, Cy5.5, Lissamine rhodamine B sulfonyl chloride, tetramethyl rhodamine isothiocyanate, rhodamine x isothiocyanate, erythrosine isothiocyanate, fluorescein, diethylenetriamine pentaacetate or other chelates of Lanthanide ions (*e.g.*, Europium, or Terbium). Donor and acceptor fluorescent moieties can be obtained, for example, from Molecular Probes (Junction City, OR) or Sigma Chemical Co. (St. Louis, MO).

The donor and acceptor fluorescent moieties can be attached to the appropriate probe oligonucleotide via a linker arm. The length of each linker arm is important, as the linker arms will affect the distance between the donor and acceptor fluorescent moieties. The length of a linker arm for the purpose of the present invention is the distance in Angstroms (Å) from the nucleotide base to the fluorescent moiety. In general, a linker arm is from about 10 to about 25 Å. The linker arm may be of the kind described in WO 84/03285. WO 84/03285 also discloses methods for attaching linker arms to a particular nucleotide base, and also for attaching fluorescent moieties to a linker arm.

An acceptor fluorescent moiety such as an LC^{™}-Red 640-NHS-ester can be combined with C6-Phosphoramidites (available from ABI (Foster City, CA) or Glen Research (Sterling, VA)) to produce, for example, LC^{™}-Red 640-Phosphoramidite. Frequently used linkers to couple a donor fluorescent moiety such as fluorescein to an oligonucleotide include thiourea linkers (FITC-derived, for example, fluorescein-CPG's from Glen Research or ChemGene (Ashland, MA)), amide-linkers (fluorescein-NHS-ester-derived, such as fluorescein-CPG from BioGenex (San Ramon, CA)), or 3'-amino-CPG's that require coupling of a fluorescein-NHS-ester after oligonucleotide synthesis.

### Detection of Variola virus

Conventional PCR methods have been used to detect variola virus. Conventional PCR-based amplification is generally followed by transfer of the amplification products to a solid support and detection using a labeled probe (*e.g*., a Southern or Northern blot). These methods are labor intensive and frequently require more than one day to complete. Additionally, the manipulation of amplification products for the purposes of detection (*e.g*., by blotting) increases the risk of carry-over contamination and false positives. By using commercially available real-time PCR instrumentation (*e.g*., LightCycler^{™}, Roche Molecular Biochemicals, Indianapolis, IN), PCR amplification and detection of the amplification product can be combined in a single closed cuvette with dramatically reduced cycling time. Since detection occurs concurrently with amplification, the real-time PCR methods obviate the need for manipulation of the amplification product, and diminish the risk of cross-contamination between amplification products. Real-time PCR greatly reduces turn-around time and is an attractive alternative to conventional PCR techniques in the clinical laboratory.

The present invention provides methods for detecting the presence or absence of variola virus in a biological sample from an individual. Methods provided by the invention avoid problems of sample contamination, false negatives, and false positives. The methods include performing at least one cycling step that includes contacting a sample with a pair of HA primers. If an HA nucleic acid molecule from variola virus is present in the biological sample, an HA amplification product is produced. Each of the HA primers anneals to a target within or adjacent to a variola virus HA nucleic acid molecule such that at least a portion of the amplification product contains nucleic acid sequence corresponding to HA and, more importantly, such that the amplification product contains the nucleic acid sequences that are complementary to the HA probes. The HA amplification product is produced provided that variola virus nucleic acid is present. Each cycling step further includes contacting the sample with a pair of HA probes. According to the invention, one of the HA probes is labeled with a donor fluorescent moiety and the other is labeled with a corresponding acceptor fluorescent moiety. The presence or absence of FRET between the donor fluorescent moiety of the first HA probe and the corresponding acceptor fluorescent moiety of the second HA probe is detected.

Each cycling step includes an amplification step and a hybridization step, and each cycling step is usually followed by a FRET detecting step. Multiple cycling steps are performed, preferably in a thermocycler. The above-described methods for detecting variola virus in a biological sample using primers and probes directed toward HA also can be performed using other variola virus gene-specific primers and probes TK-specific primers and TK-specific probes.

As used herein, "amplifying" refers to the process of synthesizing nucleic acid molecules that are complementary to one or both strands of a template nucleic acid molecule (*e.g*., variola virus HA or TK nucleic acid molecules). Amplifying a nucleic acid molecule typically includes denaturing the template nucleic acid, annealing primers to the template nucleic acid at a temperature that is below the melting temperatures of the primers, and enzymatically elongating from the primers to generate an amplification product. Amplification typically requires the presence of deoxyribonucleoside triphosphates, a DNA polymerase enzyme (*e.g*., Platinum® Taq) and an appropriate buffer and/or co-factors for optimal activity of the polymerase enzyme *(e.g.,* MgCl₂ and/or KCl).

If amplification of variola virus nucleic acid occurs and an amplification product is produced, the step of hybridizing results in a detectable signal based upon FRET between the members of the pair of probes. As used herein, "hybridizing" refers to the annealing of probes to an amplification product. Hybridization conditions typically include a temperature that is below the melting temperature of the probes but that avoids nonspecific hybridization of the probes.

Generally, the presence of FRET indicates the presence of variola virus in the biological sample, and the absence of FRET indicates the absence of variola virus in the biological sample. Inadequate specimen collection, transportation delays, inappropriate transportation conditions, or use of certain collection swabs (calcium alginate or aluminum shaft) are all conditions that can affect the success and/or accuracy of a test result, however.

Using the methods disclosed herein, detection of FRET within 30 cycling steps is indicative of a variola virus infection. Samples in which FRET is detected after more than 30 cycling steps also is indicative of a variola virus infection, but can be evaluated for variola virus infection, if desired, using a method of the invention with a different gene target or an assay other than the real-time PCR described herein. The cycle number at which FRET is detectable can be correlated with the amount of variola virus in a biological sample and, hence, in the individual (*e.g.,* viral load).

Methods of the invention also can be used for variola virus vaccine efficacy studies or epidemiology studies. For example, an attenuated variola virus in a variola vaccine can be detected using the methods of the invention during the time when virus is still present in an individual. For such vaccine efficacy studies, the methods of the invention can be used to determine, for example, the replicating ability or persistence of an attenuated virus used in a vaccine, or can be performed in conjunction with an additional assay such as a serologic assay to monitor an individual's immune response to such a vaccine. In addition, methods of the invention can be used to distinguish one variola virus strain from another for epidemiology studies of, for example, the origin or severity of an outbreak of variola (smallpox).

Representative biological samples that can be used in practicing the methods of the invention include dermal swabs, cerebrospinal fluid, ganglionic tissue, brain tissue, ocular fluid, blood, sputum, bronchio-alveolar lavage, bronchial aspirates, lung tissue, and urine. Biological sample collection and storage methods are known to those of skill in the art. Biological samples can be processed (*e.g.,* by nucleic acid extraction methods and/or kits known in the art) to release variola virus nucleic acid or in some cases, the biological sample is contacted directly with the PCR reaction components and the appropriate oligonucleotides.

Melting curve analysis is an additional step that can be included in a cycling profile. Melting curve analysis is based on the fact that DNA melts at a characteristic temperature called the melting temperature (Tm), which is defined as the temperature at which half of the DNA duplexes have separated into single strands. The melting temperature of a DNA depends primarily upon its nucleotide composition. Thus, DNA molecules rich in G and C nucleotides have a higher Tm than those having an abundance of A and T nucleotides. By detecting the temperature at which signal is lost, the melting temperature of probes can be determined. Similarly, by detecting the temperature at which signal is generated, the annealing temperature of probes can be determined. The melting temperature(s) of the HA or TK probes from the respective amplification product can confirm the presence or absence of variola virus in the sample.

Within each thermocycler run, control samples are cycled as well. Positive control samples can amplify variola virus nucleic acid control template (other than HA or TK) using, for example, control primers and control probes. Positive control samples can also amplify, for example, a plasmid construct containing variola virus HA or TK nucleic acid molecule. Such a plasmid control can be amplified internally (*e.g.*, within the biological sample) or in a separate sample run side-by-side with the patients' samples. Each thermocycler run should also include a negative control that, for example, lacks variola virus template DNA. Such controls are indicators of the success or failure of the amplification, hybridization and/or FRET reaction. Therefore, control reactions can readily determine, for example, the ability of primers to anneal with sequence-specificity and to initiate elongation, as well as the ability of probes to hybridize with sequence-specificity and for FRET to occur.

In an embodiment, the methods of the invention include steps to avoid contamination. For example, an enzymatic method utilizing uracil-DNA glycosylase is described in U.S. Patent Nos. 5,035,996, 5,683,896 and 5,945,313 to reduce or eliminate contamination between one thermocycler run and the next. In addition, standard laboratory containment practices and procedures are desirable when performing methods of the invention. Containment practices and procedures include, but are not limited to, separate work areas for different steps of a method, containment hoods, barrier filter pipette tips and dedicated air displacement pipettes. Consistent containment practices and procedures by personnel are necessary for accuracy in a diagnostic laboratory handling clinical samples.

Conventional PCR methods in conjunction with FRET technology can be used to practice the methods of the invention. In one embodiment, a LightCycler^{™} instrument is used. A detailed description of the LightCycler^{™} System and real-time and on-line monitoring of PCR can be found at http://biochem.roche.com/lightcycler. The following patent applications describe real-time PCR as used in the LightCycler^{™} technology: WO 97/46707, WO 97/46714 and WO 97/46712. The LightCycler^{™} instrument is a rapid thermal cycler combined with a microvolume fluorometer utilizing high quality optics. This rapid thermocycling technique uses thin glass cuvettes as reaction vessels. Heating and cooling of the reaction chamber are controlled by alternating heated and ambient air. Due to the low mass of air and the high ratio of surface area to volume of the cuvettes, very rapid temperature exchange rates can be achieved within the LightCycler^{™} thermal chamber. Addition of selected fluorescent dyes to the reaction components allows the PCR to be monitored in real time and on-line. Furthermore, the cuvettes serve as an optical element for signal collection (similar to glass fiber optics), concentrating the signal at the tip of the cuvette. The effect is efficient illumination and fluorescent monitoring of microvolume samples.

The LightCycler^{™} carousel that houses the cuvettes can be removed from the instrument. Therefore, samples can be loaded outside of the instrument (in a PCR Clean Room, for example). In addition, this feature allows for the sample carousel to be easily cleaned and sterilized. The fluorometer, as part of the LightCycler^{™} apparatus, houses the light source. The emitted light is filtered and focused by an epi-illumination lens onto the top of the cuvette. Fluorescent light emitted from the sample is then focused by the same lens, passed through a dichroic mirror, filtered appropriately, and focused onto data-collecting photohybrids. The optical unit currently available in the LightCycler^{™} instrument (Roche Molecular Biochemicals, Catalog No. 2 011 468) includes three bandpass filters (530 nm, 640 nm, and 710 nm), providing three-color detection and several fluorescence acquisition options. Data collection options include once per cycling step monitoring, fully continuous single-sample acquisition for melting curve analysis, continuous sampling (in which sampling frequency is dependent on sample number) and/or stepwise measurement of all samples after defined temperature interval.

The LightCycler^{™} can be operated using a PC workstation and can utilize a Windows NT operating system. Signals from the samples are obtained as the machine positions the capillaries sequentially over the optical unit. The software can display the fluorescence signals in real-time immediately after each measurement. Fluorescent acquisition time is 10-100 milliseconds (msec). After each cycling step, a quantitative display of fluorescence vs. cycle number can be continually updated for all samples. The data generated can be stored for further analysis.

As an alternative to FRET, an amplification product can be detected using a double-stranded DNA binding dye such as a fluorescent DNA binding dye (*e.g.,* SYBRGreenI® or SYBRGoId® (Molecular Probes)). Upon interaction with the double-stranded nucleic acid, such fluorescent DNA binding dyes emit a fluorescence signal after excitation with light at a suitable wavelength. A double-stranded DNA binding dye such as a nucleic acid intercalating dye also can be used. When double-stranded DNA binding dyes are used, a melting curve analysis is usually performed for confirmation of the presence of the amplification product.

As described herein, amplification products can be detected using labeled hybridization probes that take advantage of FRET technology. A common format of FRET technology utilizes two hybridization probes. Each probe can be labeled with a different fluorescent moiety and are generally designed to hybridize in close proximity to each other in a target DNA molecule (*e.g*., an amplification product). A donor fluorescent moiety, for example, fluorescein, is excited at 470 nm by the light source of the LightCycler^{™} Instrument. During FRET, the fluorescein transfers its energy to an acceptor fluorescent moiety such as LightCycler^{™}-Red 640 (LC^{™}-Red 640) or LightCycler^{™}-Red 705 (LC^{™-}Red 705). The acceptor fluorescent moiety then emits light of a longer wavelength, which is detected by the optical detection system of the LightCycler^{™} instrument. Efficient FRET can only take place when the fluorescent moieties are in direct local proximity and when the emission spectrum of the donor fluorescent moiety overlaps with the absorption spectrum of the acceptor fluorescent moiety. The intensity of the emitted signal can be correlated with the number of original target DNA molecules (*e.g*., the number of virus particles).

Another FRET format utilizes TaqMan® technology to detect the presence or absence of an amplification product, and hence, the presence or absence of Variola virus. TaqMan® technology utilizes one single-stranded hybridization probe labeled with two fluorescent moieties. When the first fluorescent moiety is excited with light of a suitable wavelength, the absorbed energy is transferred to the second fluorescent moiety according to the principles of FRET. The second fluorescent moiety is generally a quencher molecule. During the annealing step of the PCR reaction, the labeled hybridization probe binds to the target DNA (*i.e.,* the amplification product) and is degraded by the 5'→3' exonuclease activity of a polymerase (*e.g*., Taq Polymerase® ) during the subsequent elongation phase. As a result of degradation, the excited fluorescent moiety and the quencher moiety become spatially separated from one another. As a consequence, upon excitation of the first fluorescent moiety in the absence of the quencher, fluorescence emission from the first fluorescent moiety can be detected. By way of example, an ABI PRISM® 7700 Sequence Detection System (Applied Biosystems, Foster City, CA) uses TaqMan® technology, and is suitable for performing the methods described herein for detecting Variola virus. Information on PCR amplification and detection using an ABI PRISM® 770 system can be found at http://www.appliedbiosystems.com/products.

Molecular beacons in conjunction with FRET also can be used to detect the presence of an amplification product using the real-time PCR methods of the invention. Molecular beacon technology uses a single hybridization probe labeled with a first fluorescent moiety and a second fluorescent moiety. The second fluorescent moiety is generally a quencher, and the fluorescent labels are typically located at each end of the probe. Molecular beacon technology uses a probe oligonucleotide having sequences that permit secondary structure formation *(e.g.,* a hairpin). As a result of secondary structure formation within the probe, both fluorescent moieties are in spatial proximity when the probe is in solution. After hybridization to the desired amplification product(s), the secondary structure of the probe is disrupted and the fluorescent moieties become separated from one another such that after excitation with light of a suitable wavelength, the emission of the first fluorescent moiety can be detected.

It is understood that the present invention is not limited by the configuration of one or more commercially available instruments.

### Articles of manufacture

An article of manufacture can include primers and probes used to detect variola virus, together with suitable packaging materials. Representative primers and probes for detection of variola virus are capable of hybridizing to variola virus HA or TK nucleic acid molecules. Methods of designing primers and probes are disclosed herein, and representative examples of primers and probes that amplify and hybridize to variola virus HA or TK nucleic acid molecules are provided. Articles of manufacture also can include one or more fluorscent moieties for labeling the probes or, alternatively, the probes supplied with the kit can be labeled. For example, an article of manufacture may include a donor fluorescent moiety for labeling one of the HA or TK probes and an acceptor fluorescent moiety for labeling the other HA or TK probe. Examples of suitable FRET donor fluorescent moieties and corresponding acceptor fluorescent moieties are provided above.

Articles of manufacture also can contain a package insert or package label having instructions thereon for using the HA primers and probes or TK primers and probes to detect variola virus in a biological sample. Articles of manufacture may additionally include reagents for carrying out the methods disclosed herein (*e.g.*, buffers, polymerase enzymes, co-factors, or agents to prevent contamination). Such reagents may be specific for one of the commercially available instruments described herein.

The invention will be further described in the following examples, which do not limit the scope of the invention described in the claims.

### EXAMPLES

### Example 1-Specimens and Nucleic Acid Extractions

Nucleic acid was extracted from HSV-1; HSV-2; VZV; and Vaccinia (IsoQuick, Orca Research, Inc., Bothell, WA) for use in real-time PCR assays. An intact plasmid (CDC-HA1) containing a portion of the HA gene from variola virus (approximately nucleotides 15,000-15,300 of GenBank Accession No. X65516 and NC 001611) also was used in real time PCR assays.

The sample and an equal volume of lysis buffer were placed in a 1.5 ml microcentrifuge tube. 700 µl of extraction matrix and 400 µl of extraction buffer were added and the tube was centrifuged for 5 min at 13,000 rpm. The top aqueous layer was placed in a fresh tube and 1/10 volume of sodium acetate, 2 µl of glycogen, and an equal volume of isopropyl alcohol was added. The tube was then centrifuged for 10 min at 13,000 rpm. The alcohol was poured off and 2 volumes of 70% ethanol added; the tube was then centrifuged for 5 min at 13,000 rpm. The ethanol was aspirated from the tube and the pellet resuspended in 100 µl of RNase-free water.

### Example 2―LightCycler^{™} PCR

The LightCycler^{™} instrument can amplify target nucleic acids within about 30-40 min and monitors the development of PCR product by fluorescence assay after each cycling step (amplification and hybridization). All samples are amplified by LightCycler^{™} PCR with primers directed to both hemagglutinin (HA) and thymidine kinase (TK). A first set of primers and probes is directed to a first portion of the HA (HA1) gene of vaccinia virus, while a second set of primers and probes is directed to a second portion of the HA (HA2) gene of vaccinia virus. The nucleotide sequence of vaccinia virus HA is homologous with HA from the smallpox virus. A third set of primers and probes is directed to the TK gene of vaccinia virus. The nucleotide sequences of vaccinia virus TK is homologous with TK from the smallpox virus with the exception of two base pairs. Smallpox virus can be differentiated from vaccinia virus based on the melting curve of the probes from the respective amplification product.

PCR primers for detection of variola virus DNA directed to HA were designed using the OLIGO program. The primers and probes directed to a first portion of HA (HA1) had the following sequences: sense, 5'-CTA ATA TCA TTA GTA TAC GCT ACA C-3' (SEQ ID NO: 1); antisense, 5'-GAG TCG TAA GAT ATT TTA TCC-3' (SEQ ID NO:2); and probes 5'-AAT GAT TAT GTT GTT ATG AGT GCT TG-fluorescein-3' (SEQ ID NO: 3) and 5'-Red 640-TAT AAG GAG CCC AAT TCC ATT ATT CT-phosphate-3' (SEQ ID NO:4). Amplification of variola virus DNA using HA1 primers generated a 204 bp amplification product. The primers and probes directed to a second portion of HA (HA2) had the following sequences: sense, 5'-ATA GTG AAT CGA CTA TAG ACA TAA TA-3' (SEQ ID NO:5); antisense, 5'-TTG ATT TAG TAG TGA CAA TTC C-3' (SEQ ID NO:6); and probes 5'-CTG TCA CAT ACA CTA GTG ATA TCA TT-fluorescein-3' (SEQ ID NO:7); and 5'-Red 640-ATA CAG TAA GTA CAT CAT CTG GAG AA-phosphate-3' (SEQ ID NO: 8). Amplification of variola virus DNA using HA2 primers generated a 326 bp amplification product.

Primers and probes for detection of variola virus DNA using TK were designed using the OLIGO software (Molecular Biology Insights, Inc., Cascade, CO) and had the following sequences: sense, 5'-AAG GAC AGT TCT TTC CAG-3' (SEQ ID NO:9); antisense, 5'-TGA TAC ATA TCA TTA CCT CCT A-3' (SEQ ID NO: 10); and probes 5'-CCG TTT AAT AAT ATC TTG GAT CTT-fluorescein-3' (SEQ ID NO:11); and 5'-Red 640-TTC CAT TAT CTG AAA TGG TGG T-phosphate-3' (SEQ ID NO:12). Alternatively, TK probes having the following sequences were used to detect the TK amplification product: 5'-GAC ATT TCA ACG TAA ACC GTT TAA-fluorescein-3' (SEQ ID NO: 13) and 5'-Red 640-AAT ATC TTG GAT CTT ATT CCA TTA TCT G-phosphate-3' (SEQ ID NO: 14). Amplification of variola virus DNA using such TK primers produced an amplification product of 250 bp.

Each set of hybridization probes (*i.e*., HA and the TK probes) contained a donor fluorophore (fluorescein) on the 3'-end of one probe, which when excited by an external light source, emitted light that was absorbed by a corresponding acceptor fluorophore (LC-Red 640) at the 5'-end of the second hybridization probe.

For the real-time PCR assay, 5 µl of each nucleic acid sample is added to 15 µl of a PCR Master Mix in each reaction capillary. A no-template control receives 15 µl of reaction mixture with 5 µl water. PCR Master Mix is optimized for the LightCycler^{™} and contains the following: 0.2 mM of deoxyribonucleoside triphosphates (50 mM KCl, 10 mM Tris-Cl (pH 8.3)), 4 mM MgCl₂, 0.7 µM primers, 0.025% bovine serum albumin (BSA), 0.2 µM fluorescein-probe, 0.4 µM LC-Red 640-probe, 2% DMSO, 0.2% uracil-DNA glycosylase, and 0.03 units/ml of platinum *Taq* (Perkin-Elmer Corp., Branchburg, NJ). The PCR reagents and specimen extract are centrifuged in the capillary to facilitate mixing. All capillaries are then sealed and placed in the LightCycler apparatus.

Samples are initially treated at about 37°C for about 5 min in the presence of uracil-DNA glycosylase. The uracil-DNA glycosylase is then inactivated by heating the samples for about 3 min at about 95°C, after which the samples undergo 45 cycles of: denaturation at about 95°C for 10 secs followed by primer annealing to the template nucleic acid for about 15 secs at about 55°C (during which time signal is collected), and elongation of the newly-synthesized strands at about 72°C for about 15 secs. The melting curve is then determined by heating to about 95°C followed immediately by about 1 min at about 45°C. The temperature is then increased up to about 78°C at a rate of about 0.2°C per second (during which time signal is collected). The sample is then held at about 40°C for about 30 sec.

### Example 3-Autoclaved Samples

Current tests for variola (*e.g*., cell cultures, or morphological recognition by electron microscopy) require Biosafety Level-4 (BS-4) facilities. Autoclaving the biological samples prior to analysis would allow BS-2 facilities to perform variola assays. Nucleic acids from viruses related to variola (*e.g*., HSV, and Vaccinia) were examined to confirm the viability of viral nucleic acid for PCR amplification following autoclaving (*e.g*., 121 °C; 15 min; 20 psi).

Mock specimens were seeded with Vaccinia virus (VV), HSV, or VZV. A total of 30 culturette swabs were seeded with five 10-fold dilutions of each virus suspension (VV, HSV, VZV). Culturettes were placed into glass vials and sealed with screw-cap lids. Fifteen of the samples were autoclaved; the other 15 were placed at room temperature. Subsequently, all culturettes were placed into 2 ml of serum-free media. Nucleic acid extracts from all specimens (200 ^{c}cl) were assayed by LightCycler PCR specific for each virus target. Similarly, all specimens (200 ^{c}cl) were inoculated into MRC-5 tube cell cultures, incubated at 36°C, and examined daily for 5 (VV, HSV) or 10 (VZV) days for the presence of characteristic cytopathic effects. PCR conditions for detecting VV, HSV, and VZV with or without autoclaving were as described above in Example 2, except that the maximal temperature for melting curve determination was 80°C.

Autoclaving eliminated the infectivity of all three viruses as shown in Tables 1 and 2. DNA from all three viruses was detected by LightCycler PCR in both autoclaved (VV, 7/15; HSV, 11/15; VZV, 10/15) and nonautoclaved (VV, 6/15; HSV, 9/15; VZV, 8/15) samples. HSV and VV specimens (nonautoclaved) detected by LightCycler PCR also were detected in cell cultures.

**Table 1. Detection of HSV DNA With and Without Autoclaving**

| Procedure | | | LightCycler PCR results | | Infectivity in Cell Cultures |
|---|---|---|---|---|---|
| No. Samples | Autoclaved | Nucleic Acids Extracted | Pos | Neg | |
| 15 | Yes | Yes | 11 | 4 | 0 |
| 15 | No | Yes | 9 | 6 | 9 |

**Table 2. Detection of Vaccinia DNA With and Without Autoclaving**

| Procedure | | | LightCycler PCR results | | Infectivity in Cell Cultures |
|---|---|---|---|---|---|
| No. Samples | Autoclaved | Nucleic Acids Extracted | Pos | Neg | |
| 15 | Yes | Yes | 7 | 8 | 0 |
| 15 | No | Yes | 6 | 9 | 6 |

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. A method for detecting the presence or absence of variola virus in a biological sample from an individual, said method comprising:
performing at least one cycling step, wherein a cycling step comprises an amplifying step and a hybridizing step, wherein said amplifying step comprises contacting said sample with a pair of hemagglutinin (HA) primers to produce an HA amplification product if a variola virus HA nucleic acid molecule is present in said sample, wherein said hybridizing step comprises contacting said sample with a pair of HA probes, wherein the members of said pair of HA probes hybridize within no more than five nucleotides of each other, wherein a first HA probe of said pair of HA probes is labeled with a donor fluorescent moiety and said second HA probe of said pair of HA probes is labeled with a corresponding acceptor fluorescent moiety; and
detecting the presence or absence of fluorescence resonance energy transfer (FRET) between said donor fluorescent moiety of said first HA probe and said acceptor fluorescent moiety of said second HA probe,
wherein the presence of FRET is indicative of the presence of variola virus in said biological sample, and wherein the absence of FRET is indicative of the absence of variola virus in said biological sample,
further comprising the steps of
performing at least one cycling step, wherein said cycling step comprises an amplifying step and a hybridizing step, wherein said amplifying step comprises contacting said sample with a pair of thymidine kinase (TK) primers to produce a TK amplification product if a variola virus TK nucleic acid molecule is present in said sample, wherein said hybridizing step comprises contacting said sample with a pair of TK probes, wherein the members of said pair of TK probes hybridize within no more than five nucleotides of each other, wherein a first TK probe of said pair of TK probes is labeled with a donor fluorescent moiety and said second TK probe of said pair of TK probes is labeled with a corresponding acceptor fluorescent moiety; and
detecting the presence or absence of FRET between said donor fluorescent moiety of said first TK probe and said acceptor fluorescent moiety of said second TK probe.

2. The method of claim 1, wherein said biological sample is selected from the group consisting of dermal swabs, cerebrospilal fluid, ganglionic tissue, brain tissue, ocular fluid, blood, sputum, bronchio-alveolar lavage, bronchial aspirates, lung tissue, and urine.

3. The method of claim 1, wherein said cycling step is performed on a control sample.

## Patentansprüche

1. Verfahren zum Nachweis des Vorhandenseins bzw. der Abwesenheit von Variolavirus in einer biologischen Probe aus einem Individuum, wobei man in dem Verfahren:
mindestens einen Zyklusschritt durchführt, wobei ein Zyklusschritt einen Amplifikationsschritt und einen Hybridisierungsschritt umfaßt, wobei man in dem Amplifikationsschritt die Probe mit einem Paar Hämagglutinin (HA)-Primern in Kontakt bringt, so daß bei Vorhandensein eines Variolavirus-HA-Nukleinsäuremoleküls in der Probe ein HA-Amplifikationsprodukt gebildet wird, wobei man in dem Hybridisierungsschritt die Probe mit einem Paar HA-Sonden in Kontakt bringt, wobei die Mitglieder des HA-Sondenpaars nicht mehr als fünf Nukleotide voneinander entfernt hybridisieren, wobei eine erste HA-Sonde des HA-Sondenpaars mit einer Donor-Fluoreszenzgruppierung und die zweite HA-Sonde des HA-Sondenpaars mit einer entsprechenden Akzeptor-Fluoreszenzgruppierung markiert ist; und
das Vorhandensein bzw. die Abwesenheit von Fluoreszenzresonanz-Energietransfer (FRET) zwischen der Donor-Fluoreszenzgruppierung der ersten HA-Sonde und der Akzeptor-Fluoreszenzgruppierung der zweiten HA-Sonde nachweist,
wobei das Vorhandensein von FRET das Vorhandensein von Variolavirus in der biologischen Probe anzeigt und wobei die Abwesenheit von FRET die Abwesenheit von Variolavirus in der biologischen Probe anzeigt,
wobei man in weiteren Schritten
mindestens einen Zyklusschritt durchführt, wobei ein Zyklusschritt einen Amplifikationsschritt und einen Hybridisierungsschritt umfaßt, wobei man in dem Amplifikationsschritt die Probe mit einem Paar Thymidinkinase (TK)-Primern in Kontakt bringt, so daß bei Vorhandensein eines Variolavirus-TK-Nukleinsäuremoleküls in der Probe ein TK-Amplifikationsprodukt gebildet wird, wobei man in dem Hybridisierungsschritt die Probe mit einem Paar TK-Sonden in Kontakt bringt, wobei die Mitglieder des TK-Sondenpaars nicht mehr als fünf Nukleotide voneinander entfernt hybridisieren, wobei eine erste TK-Sonde des TK-Sondenpaars mit einer Donor-Fluoreszenzgruppierung und die zweite TK-Sonde des TK-Sondenpaars mit einer entsprechenden Akzeptor-Fluoreszenzgruppierung markiert ist; und
das Vorhandensein bzw. die Abwesenheit von FRET zwischen der Donor-Fluoreszenzgruppierung der ersten TK-Sonde und der Akzeptor-Fluoreszenzgruppierung der zweiten TK-Sonde nachweist.

2. Verfahren nach Anspruch 1, wobei die biologische Probe ausgewählt ist aus der Gruppe: Hautabstriche, Liquor, Gangliongewebe, Hirngewebe, Augenflüssigkeit, Blut, Speichel, bronchoalveoläre Lavage, Bronchialaspirate, Lungengewebe und Urin.

3. Verfahren nach Anspruch 1, wobei der Zyklusschritt an einer Kontrollprobe durchgeführt wird.

## Revendications

1. Procédé destiné à détecter la présence ou l'absence du virus de la variole dans un échantillon biologique d'un individu, ledit procédé comprenant :
la réalisation d'au moins un cycle, dans lequel un cycle comprend une étape d'amplification et une étape d'hybridation, dans lequel ladite étape d'amplification comprend la mise en contact dudit échantillon avec une paire d'amorces d'hémagglutinine (HA) destinés à produire un produit d'amplification HA dans le cas où une molécule d'acide nucléique HA du virus de la variole est présente dans ledit échantillon, dans lequel ladite étape d'hybridation comprend la mise en contact dudit échantillon avec une paire de sondes HA, dans lequel les membres de ladite paire de sondes HA s'hybrident à une distance de maximum cinq nucléotides l'un de l'autre, dans lequel une première sonde HA de ladite paire de sondes HA est marquée au moyen d'un groupement fluorescent donneur et ladite seconde sonde HA de ladite paire de sondes HA est marquée au moyen d'un groupement fluorescent accepteur correspondant ; et
la détection de la présence ou de l'absence de transfert d'énergie de résonance par fluorescence (FRET) entre ledit groupement fluorescent donneur de ladite première sonde HA et ledit groupement fluorescent accepteur de ladite seconde sonde HA,
dans laquelle la présence de FRET indique la présence du virus de la variole dans ledit échantillon biologique, et dans laquelle l'absence de FRET indique l'absence du virus de la variole dans ledit échantillon biologique, comprenant en outre les étapes consistant en
la réalisation d'au moins un cycle, dans lequel un cycle comprend une étape d'amplification et une étape d'hybridation, dans lequel ladite étape d'amplification comprend la mise en contact dudit échantillon avec une paire d'amorces de thymidine kinase (TK) destinés à produire un produit d'amplification TK dans le cas où une molécule d'acide nucléique TK du virus de la variole est présente dans ledit échantillon, dans lequel l'étape d'hybridation comprend la mise en contact dudit échantillon avec une paire de sondes TK, dans lequel les membres de ladite paire de sondes TK s'hybrident à une distance de maximum cinq nucléotides l'un de l'autre, dans lequel une première sonde TK de ladite paire de sondes TK est marquée au moyen d'un groupement fluorescent donneur et ladite seconde sonde TK de ladite paire de sondes TK est marquée au moyen d'un groupement fluorescent accepteur correspondant ; et
la détection de la présence ou de l'absence de FRET entre ledit groupement fluorescent donneur de ladite première sonde TK et ledit groupement fluorescent accepteur de ladite seconde sonde TK.

2. Procédé selon la revendication 1, dans lequel l'échantillon biologique est sélectionné parmi le groupe composé de prélèvements dermiques, de liquide cérébrospinal, de tissu ganglionnaire, de tissu cérébral, de liquide oculaire, de sang, d'expectoration, de lavage bronchio-alvéolaire, d'échantillons prélevés par aspiration bronchique, de tissu pulmonaire et d'urine.

3. Procédé selon la revendication 1, dans lequel le cycle est réalisé sur un échantillon témoin.


### SEQUENCE LISTING

<110> Roche Diagnostics GmbH
   Mayo Foundation for Medical Education and Research
<120> Detection of Variola Virus
<130> 21434 EP
<140>
   <141>
<150> US 60/338,237
   <151> 2001-11-02
<150> US 60/341,601
   <151> 2001-12-17
<160> 14
<170> PatentIn Ver. 2.1
<210> 1
   <211> 25
   <212> DNA
   <213> Artificial Sequence
<220>
   <223> Description of Artificial Sequence:primer
<400> 1
   ctaatatcat tagtatacgc tacac 25
<210> 2
   <211> 21
   <212> DNA
   <213> Artificial Sequence
<220>
   <223> Description of Artificial Sequence:primer
<400> 2
   gagtcgtaag atattttatc c 21
<210> 3
   <211> 26
   <212> DNA
   <213> Artificial Sequence
<220>
   <223> Description of Artificial Sequence:primer
<400> 3
   aatgattatg ttgttatgag tgcttg 26
<210>4
   <211> 26
   <212> DNA
   <213> Artificial Sequence
<220>
   <223> Description of Artificial Sequence:primer
<400> 4
   tataaggagc ccaattccat tattct 26
<210> 5
   <211> 26
   <212> DNA
   <213> Artificial Sequence
<220>
   <223> Description of Artificial Sequence:primer
<400> 5
   atagtgaatc gactatagac ataata 26
<210> 6
   <211> 22
   <212> DNA
   <213> Artificial Sequence
<220>
   <223> Description of Artificial Sequence:primer
<400> 6
   ttgatttagt agtgacaatt cc 22
<210> 7
   <211> 26
   <212> DNA
   <213> Artificial Sequence
<220>
   <223> Description of Artificial Sequence:primer
<400> 7
   ctgtcacata cactagtgat atcatt 26
<210> 8
   <211> 26
   <212> DNA
   <213> Artificial Sequence
<220>
   <223> Description of Artificial Sequence:primer
<400> 8
   atacagtaag tacatcatct ggagaa 26
<210> 9
   <211> 18
   <212> DNA
   <213> Artificial Sequence
<220>
   <223> Description of Artificial Sequence:primer
<400> 9
   aaggacagtt ctttccag 18
<210> 10
   <211> 22
   <212> DNA
   <213> Artificial Sequence
<220>
   <223> Description of Artificial Sequence:primer
<400> 10
   tgatacatat cattacctcc ta 22
<210> 11
   <211> 24
   <212> DNA
   <213> Artificial Sequence
<220>
   <223> Description of Artificial Sequence:primer
<400> 11
   ccgtttaata atatcttgga tctt 24
<210> 12
   <211> 22
   <212> DNA
   <213> Artificial Sequence
<220>
   <223> Description of Artificial Sequence:primer
<400> 12
   ttccattatc tgaaatggtg gt 22
<210> 13
   <211> 24
   <212> DNA
   <213> Artificial Sequence
<220>
   <223> Description of Artificial Sequence:primer
<400> 13
   gacatttcaa cgtaaaccgt ttaa 24
<210> 14
   <211> 28
   <212> DNA
   <213> Artificial Sequence
<220>
<223> Description of Artificial Sequence:primer
<400> 14
   aatatcttgg atcttattcc attatctg 28